# EUROPEAN PATENT SPECIFICATION

(11) **EP 0 460 774 B1**
(45) Date of publication and mention of the grant of the patent: **07.02.1996**
(21) Application number: 91201911.4
(22) Date of filing: 18.05.1987
(51) Int. Cl.: A61L 15/28, A61L 15/40, A61L 15/46

(54) **Wound dressing**
Wundverband
Pansement pour blessure

(43) Date of publication of application: 11.12.1991
(62) Divisional of application: 87304365.7
(73) Proprietor: BRITISH TECHNOLOGY GROUP LIMITED, London SE1 6BU (GB)
(72) Inventor: Sagar, Brian, Cheadle, Cheshire SK8 4HJ (GB); Hamlyn, Paul, East Didsbury, Manchester (GB); Wales, David, Reddish, Stockport SK5 6JN (GB)
(74) Representative: Percy, Richard Keith

(56) References cited:
- GB-A- 2 148 959

## Description

This invention concerns wound dressings.

The wound healing properties of chitin and chitin derivatives have long been recognised and documented. The extraction of chitin from its natural sources and its incorporation in conventional wound dressings, however, is quite costly.

The present invention provides an improved wound dressing at economic cost.

The invention comprises a wound dressing comprised by an assembly of microfungal fibres which have been treated with alkali to expose chitin and chitosan, characterised in that the assembly is frozen and freeze-dried.

The microfungal fibres may be hyphae or sporangiophores and may for example be Mucor mucedo or Rhizomucor miehei.

The assembly may be a wet-laid mat, which may include a plasticiser which may be glycerol or polyethylene glycol, for example.

The assembly may be cut to a desired size and sealed in a water vapour impermeable pack.

The microfungal fibres may be bleached. The assembly may include other fibres of substances known to assist or facilitate wound healing, such as collagen, a well-known haemostatic agent or an alginate, useful as a physical barrier to prevent drying and adhesion between the wound and the dressing material. The assembly may also incorporate bound metallic silver, useful as an anti-bacterial agent.

The assembly may be treated with a bi-functional cross-linking agent such as glutaraldehyde.

The invention will be further apparent from the following description which concerns by way of example only the preparation of various forms of wound dressing embodying same and with reference to the accompanying drawings, in which:
- Figure 1: is a diagrammatic illustration of a batch process for preparing a first form of wound dressing;
- Figure 2: is a diagrammatic illustration of a batch process for making a second form of wound dressing; and
- Figure 3: is a diagrammatic illustration of a continuous process for making the second form of wound dressing.

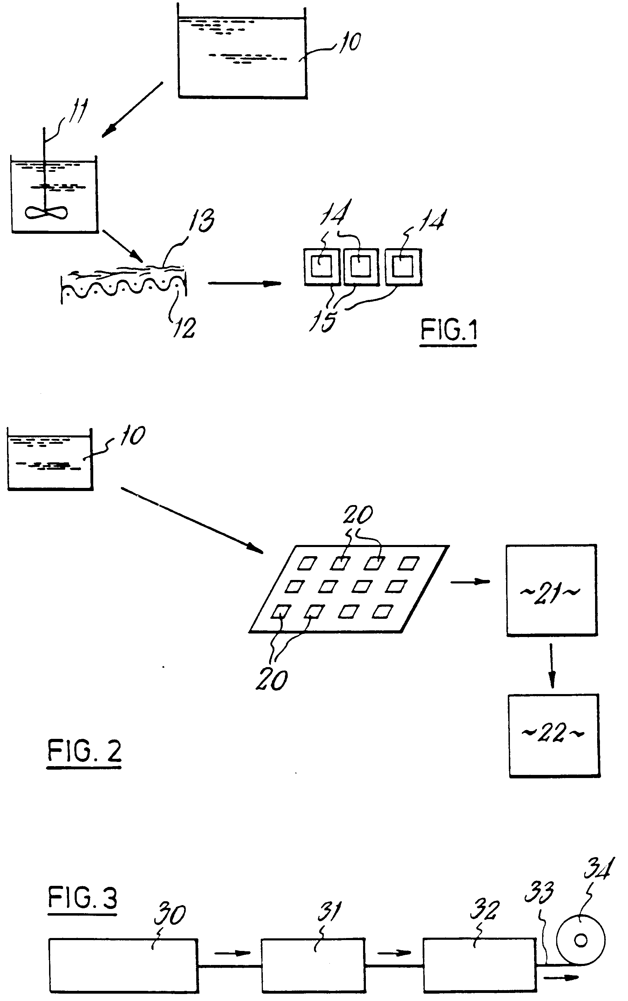

Referring firstly to Figure 1, micro-fungal mycelia are produced from a culture of Mucor mucedo (CMI 184 726), grown in a nutrient solution containing malt extract (17g/1) and mycological peptone (3g/1) in a fermenter vessel 10 at a temperature of 30^{o}C for one to two days.

The culture is then washed and treated with a 2N boiling solution of sodium hydroxide for one hour to dissolve protein from the outer layers of the cell walls and expose the underlying chitin and chitosan. Further de-acetylisation of the chitin may be effected by 40% sodium hydroxide solution.

The culture is repeatedly washed until neutral pH is obtained and then bleached by treatment with a solution of hydrogen peroxide (80 ml/l 37% H₂O₂ + 40 g/l NaOH + 40 g/l sodium silicate) for two hours at room temperature.

The culture is washed again and disintegrated using normal paper making equipment 11 to ensure an even dispersion of the hyphae in water to form a slurry. The slurry is strained through a filter medium 12 to leave a wet-laid matt 13 having a thickness of 1mm or thereabouts.

If desired other fibres having wound healing properties such as of collagen or an alginate or both may be mixed with the fibres before the matt is laid, as may textile fibres to give mechanical strength or other properties.

Suitably sized portions 14 for wound dressings of desired size are cut from the matt 13 and, according to parent European Application No.87304365.7 (0,291,587), from which this application is divided, immediately encapsulated whilst still wet in airtight packs 15 and subsequently sterilised. The retained water acts as a plasticiser to prevent the fibres from becoming dry and brittle and also ensures that the dressings are moist when removed from the packs for use.

Alternatively glycerol or polyethylene glycol may be added to the slurry before the matt is laid.

In another example Mucor mucedo is replaced by Rhizomucor miehei (CMI 147 066) which is fermented at 50^{o}C.

As shown in Figure 2, however, and according to the present invention, the slurry from vessel 10 is poured into shaped moulds or dishes 20 which are then frozen in a deep freezer 21 for, say, sixteen hours and then freeze-dried for twenty eight hours in a freeze-drier 22. Absorbent pads typically of say 10cms in diameter or larger and from a few millimetres to several centimetres thick can be produced in this way.

Figure 3 illustrates a continuous process for producing a web, in which the slurry is laid down using conventional paper making machinery 30 and passed straight into a continuous freezer 31 and from there into a continuous freeze drying plant 32 after which the resulting matt 33 can be rolled up as at 34.

It is surprising that the matt is so flexible and strong as to permit this since, previously, 100% fungal matts have been brittle unless plasticiser has been added.

Use of a placticiser in freeze-dried wound dressings is not necessary but a plasticiser may of course be added if desired.

Thus for example, the matts or pads or fibres from which they are formed may be treated in a solution of silver nitrate whereby silver ions will be captured by the chitosan and thus be present in the dressings as an anti-bacterial agent.

Again for example, the dressings may be treated with a bi-functional cross-linking agent such as glutaraldehyde.

Yet again, for example the wet-laid matts may be laminated with one or more backing layers of conventional, textile fibre if desired.

Tests carried out by Royal National Orthopaedic Hospital at Stanmore Middlesex, indicated that wound dressings prepared in accordance with the invention from Mucor mucedo gave encouraging results in terms of the quality and quantity of repair tissue.

## Claims

1. A wound dressing comprised by an assembly of microfungal fibres which have been treated with alkali to expose chitin and chitosan, characterised in that the assembly is frozen and freeze-dried.

2. A wound dressing according to claim 1, characterised in that the microfungal fibres are hyphae.

3. A wound dressing according to claim 1, characterised in that the microfungal fibres are sporangiophores.

4. A wound dressing according to any one of claims 1 to 3, characterised in that the assembly is a wet-laid mat.

5. A wound dressing according to claim 4, characterised in that the wet-laid mat includes a plasticiser.

6. A wound dressing according to any one of claims 1to 5, characterised in that the assembly is cut to size and sealed in a water vapour impermeable pack.

7. A wound dressing according to any one of claims 1 to 6, characterised in that the microfungal fibres are bleached.

8. A wound dressing according to any preceding claim, characterised in that other fibres are included with the microfungal fibres.

9. A wound dressing according to any preceding claim, characterised in that the alkali treated fibres are treated with a silver salt whereby silver ions are captured and present in the dressing as an anti-bacterial agent.

10. A wound dressing according to any preceding claim, characterised in that the assembly is treated with a bi-functional cross-linking agent.

11. A wound dressing according to any one of claims 1 to 10, characterised in that the assembly is laminated with one or more backing layers of conventional textile material.

12. A method for making a wound dressing of microfungal fibres which have been treated with alkali to expose chitin and chitosan, characterised by comprising the step of freezing and freeze drying an assembly of the fibres.

13. A method according to claim 12, characterised by forming a wet-laid mat of the fibres and freezing and freeze drying the same.

14. A method according to claim 12, characterised in that a microfungal fibre slurry is poured into shaped moulds or dishes (20), frozen in a freezer and then freeze dried.

15. A method according to claim 12, characterised in that a microfungal fibre slurry is laid down using conventional paper making machinery (30).

16. A method according to claim 15, characterised in that the laid down slurry is frozen in a continuous freezer (31).

17. A method according to claim 16, characterised in that the frozen, laid down slurry is freeze dried in a continuous freeze drying plant.

## Patentansprüche

1. Wundverband, bestehend aus einer Anordnung aus mikrofungalen Fasern, die mit Alkali behandelt worden sind, um Chitin und Chitosan freizusetzen, dadurch gekennzeichnet, daß die Anordnung gefroren und gefriergetrocknet ist.

2. Wundverband nach Anspruch 1, dadurch gekennzeichnet, daß die mikrofungalen Fasern Hyphen sind.

3. Wundverband nach Anspruch 1, dadurch gekennzeichnet, daß die mikrofungalen Fasern Sporangiophoren sind.

4. Wundverband nach einem der Ansprüche 1 bis 3, dadurch gekennzeichnet, daß die Anordnung eine naßgelegte Bahn ist.

5. Wundverband nach Anspruch 4, dadurch gekennzeichnet, daß die naßgelegte Bahn einen Weichmacher enthält.

6. Wundverband nach einem der Ansprüche 1 bis 5, dadurch gekennzeichnet, daß die Anordnung auf eine bestimmte Größe geschnitten und in einer Wasserdampf undurchlässigen Verpackung eingesiegelt ist.

7. Wundverband nach einem der Ansprüche 1 bis 6, dadurch gekennzeichnet, daß die mikrofungalen Fasern gebleicht sind.

8. Wundverband nach einem der vorangehenden Ansprüche, dadurch gekennzeichnet, daß andere Fasern zusammen mit den mikrofungalen Fasern vorhanden sind.

9. Wundverband nach einem der vorangehenden Ansprüche, dadurch gekennzeichnet, daß die mit Alkali behandelten Fasern mit einem Silbersalz behandelt worden sind, wodurch Silberionen eingefangen worden sind und als antibakterielles Mittel in dem Wundverband vorhanden sind.

10. Wundverband nach einem der vorangehenden Ansprüche, dadurch gekennzeichnet, daß die Anordnung mit einem bi-funktionellen Vernetzungsmittel behandelt worden ist.

11. Wundverband nach einem der Ansprüche 1 bis 10, dadurch gekennzeichnet, daß die Anordnung mit einer oder mehreren Unterlagen aus üblichem Textilmaterial laminiert ist.

12. Verfahren zur Herstellung eines Wundverbands aus mikrofungalen Fasern, die mit Alkali behandelt worden sind, um Chitin und Chitosan freizusetzen, dadurch gekennzeichnet, daß es die Stufen des Gefrierens und des Gefriertrocknens einer Anordnung der Fasern umfaßt.

13. Verfahren nach Anspruch 12, dadurch gekennzeichnet, daß eine naßgelegte Bahn aus den Fasern gebildet wird und diese gefroren und gefriergetrocknet wird.

14. Verfahren nach Anspruch 13, dadurch gekennzeichnet, daß eine Aufschlämmung von mikrofungalen Fasern in Formen oder Schalen (20) gegossen, in einer Gefriervorrichtung gefroren und dann gefriergetrocknet wird.

15. Verfahren nach Anspruch 12, dadurch gekennzeichnet, daß die Aufschlämmung der mikrofungalen Fasern mit Hilfe einer üblichen Vorrichtung zur Papierherstellung (30) gelegt wird.

16. Verfahren nach Anspruch 15, dadurch gekennzeichnet, daß die gelegte Aufschlämmung in einer kontinuierlichen Gefriervorrichtung (31) gefroren wird.

17. Verfahren nach Anspruch 16, dadurch gekennzeichnet, daß die gefrorene gelegte Aufschlämmung in einer kontinuierlichen Gefriertrocknungsanlage gefriergetrocknet wird.

## Revendications

1. Pansement comprenant un assemblage de fibres de champignons microscopiques qui ont été traitées avec un alcali pour exposer la chitine et le chitosane, caractérisé en ce que l'assemblage est congelé et lyophilisé.

2. Pansement selon la revendication 1, caractérisé en ce que les fibres de champignons microscopiques sont des hyphes.

3. Pansement selon la revendication 1, caractérisé en ce que les fibres de champignons microscopiques sont des sporangiophores.

4. Pansement selon l'une quelconque des revendications 1 à 3, caractérisé en ce que l'assemblage est un mat déposé à l'état humide.

5. Pansement selon la revendication 4, caractérisé en ce que le mat déposé à l'état humide comprend un plastifiant.

6. Pansement selon l'une quelconque des revendications 1 à 5, caractérisé en ce que l'assemblage est coupé à une taille voulue et scellé dans un emballage imperméable à la vapeur d'eau.

7. Pansement selon lune quelconque des revendications 1 à 6, caractérisé en ce que les fibres de champignons microscopiques sont blanchies.

8. Pansement selon l'une quelconque des revendications précédentes, caractérisé en ce que d'autres fibres sont incluses avec les fibres de champignons microscopiques.

9. Pansement selon l'une quelconque des revendications précédentes, caractérisé en ce que les fibres traitées avec un alcali sont traitées avec un sel d'argent, ce par quoi, les ions argent sont piégés et présents dans le pansement en tant qu'agent anti-bactérien.

10. Pansement selon l'une quelconque des revendications précédentes, caractérisé en ce que l'assemblage est traité par un agent de réticulation bifonctionnel.

11. Pansement selon l'une quelconque des revendications 1 à 10, caractérisé en ce que l'assemblage est stratifié avec une ou plusieurs couches support d'une matière textile conventionnelle.

12. Méthode pour préparer un pansement de fibres de champignons microscopiques qui ont été traitées avec un alcali pour exposer la chitine et le chitosane, caractérisée en ce qu'elle comprend l'étape consistant à congeler et à lyophiliser un assemblage des fibres.

13. Méthode selon la revendication 12, caractérisée par les étapes consistant à former un mat déposé à l'état humide des fibres et à congeler et à lyophiliser celui-ci.

14. Méthode selon la revendication 12, caractérisée en ce qu'une suspension de fibres de champignons microscopiques est versée dans des moules ou des cuvettes (20) mis en forme, congelée dans un congélateur puis lyophilisée.

15. Méthode selon la revendication 12, caractérisée en ce qu'une suspension de fibres de champignons microscopiques est déposée en utilisant un appareillage conventionnel de fabrication du papier (30).

16. Méthode selon la revendication 15, caractérisée en ce que la suspension déposée est congelée dans un congélateur en continu (31).

17. Méthode selon la revendication 16, caractérisée en ce que la suspension déposée et congelée est lyophilisée dans une installation de lyophilisation en continu.
